(19) Europäisches Patentamt
European Patent Office
Office européen des brevets

(11) Veröffentlichungsnummer: **0 358 938**
**A2**

(12) **EUROPÄISCHE PATENTANMELDUNG**

(21) Anmeldenummer: **89114615.1**

(22) Anmeldetag: **08.08.89**

(51) Int. Cl.5: **C07K 7/06 , A61K 39/00 , G01N 33/573**

Patentansprüche für folgenden Vertragsstaat: ES.

(30) Priorität: **12.08.88 DE 3827416**

(43) Veröffentlichungstag der Anmeldung:
**21.03.90 Patentblatt 90/12**

(84) Benannte Vertragsstaaten:
**AT BE CH DE ES FR GB IT LI LU NL SE**

(71) Anmelder: **BEHRINGWERKE Aktiengesellschaft**
**Postfach 1140**
**D-3550 Marburg 1(DE)**

(72) Erfinder: **Stüber, Werner, Dr.**
**Cölber Weg 12**
**D-3551 Lahntal(DE)**
Erfinder: **Pâques, Eric Paul, Dr.**
**Schmiedeacker 18**
**D-3550 Marburg Belgier(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Peptide mit Prourokinase-Sequenzen, Verfahren zu ihrer Herstellung und ihre Verwendung, Antikörper gegen diese Peptide und Mittel enthaltend diese Antikörper zur Bestimmung von Prourokinase.**

(57) Es werden Peptide mit Aminosäure-Sequenzen der Prourokinase sowie Verfahren zu ihrer Herstellung beschrieben. Diese Peptide werden zur Gewinnung von Antikörpern gegen diese Peptide verwendet. Diese Antikörper können zur Bestimmung der Prourokinase verwendet werden.

EP 0 358 938 A2

## Peptide mit Prourokinase-Sequenzen, Verfahren zu ihrer Herstellung und ihre Verwendung, Antikörper gegen diese Peptide und Mittel enthaltend diese Antikörper zur Bestimmung von Prourokinase

Die Erfindung betrifft Peptide mit Teilen der Aminosäuresequenz der Prourokinase, Verfahren zu ihrer Herstellung und ihre Verwendung sowie Antikörper gegen diese Peptide und deren Verwendung zur Bestimmung von Prourokinase.

Prourokinase stellt das Proenzym eines Plasminogen-Aktivators dar. Die Aufgabe der Plasminogen-Aktivatoren besteht darin, das Proenzym-Plasminogen in Plasmin umzuwandeln, welches dann proteolytisch aktiv ist. Plasmin ist in einer Reihe physiologischer und pathophysiologischer Prozesse involviert, wie die Auflösung von Thromben oder der Umbau von Geweben (Ovulation, Spermatogenese, embryonales Wachstum, Makrophagenmigration, Metastasierung). Bei der Aktivierung des Fibrinolyse-Systems wird Prourokinase (PUK) in Urokinase (UK) umgewandelt. Urokinase wirkt dann als Plasminogen-Aktivator und wandelt Plasminogen in Plasmin um. Unter normalen physiologischen Bedingungen ist Urokinase in der Blutbahn nicht nachweisbar, sondern die Prourokinase. Die Bestimmung der Prourokinase im Plasma ist von großer diagnostischer Bedeutung, da eine Abnahme der Plasma-Konzentration Auskunft über das Aktivierungsstadium des Fibrinolyse-Systems ermöglichen würde. Prourokinase ist jedoch proteolytisch nicht aktiv und somit über seine biologische Aktivität nicht nachweisbar. Der Nachweis der Pro urokinase müßte deshalb immunologisch durchgeführt werden. Die nach dem Stand der Technik vorhandenen Antikörper ermöglichen jedoch nicht zwischen PUK und UK zu unterscheiden.

Die Aufgabe der Erfindung ist deshalb die Entwicklung eines Mittels und eines Verfahrens zum gezielten Nachweis sowie auch zur Reinigung der Prourokinase.

Diese Aufgabe wurde dadurch gelöst, daß spezifische Antikörper hergestellt werden, die in der Lage sind, PUK in Gegenwart von anderen Proteinen, insbesondere Urokinase, selektiv und quantitativ zu bestimmen. Diese Antikörper werden durch Immunisierung mit Peptiden, abgeleitet aus der Prourokinasesequenz, gewonnen.

Gegenstand der Erfindung sind deshalb Peptide enthaltend 4 bis 40 Aminosäuren mit Aminosäure-Sequenzen aus der Region 140 - 180 der Prourokinase, die bevorzugt die Spaltstelle 158/159 (-Lys/Ile-) enthalten.

Solche Peptide bewirken die Induktion prourokinase-spezifischer Antikörper, wenn Tiere mit einem dieser Peptide immunisiert werden.

Diese neuen Peptide können zusätzlich reaktive Gruppen, vorzugsweise am C- oder N-Terminus, beispielsweise Thiolgruppen enthalten, um gegebenenfalls an Trägermoleküle immobilisiert zu werden.

Besonders geeignet sind die Peptide (die Abkürzungen sind weiter hinten erläutert)

R P R F K I I G G E F T, L R P R F K I I G G E F T T und
G Q K T L R P R F K I I G G E F T T I E,

die gegebenenfalls am N-Terminus bzw. C-Terminus Cystein mit einer freien Sulfhydrylgruppe enthalten.

Gegenstand der Erfindung sind auch Herstellungsverfahren der erfindungsgemäßen Peptide.

Gegenstand der Erfindung ist besonders ein an sich bekanntes Verfahren zur Herstellung eines erfindungsgemäßen Peptids, dadurch gekennzeichnet, daß geschützte Aminosäurederivate oder Peptidsegmente in Lösung oder an einer Festphase aneinander gekoppelt werden und durch Abspalten der Schutzgruppen sowie im Falle einer Festphase durch Abspaltung vom Trägerharz ein erfindungsgemäßes Peptid erhalten wird.

Ganz besonders bevorzugt ist das mit dieser Technik aufgebaute Peptid Cys-Leu-Arg-Pro-Arg-Phe-Lys-Ile-Ile-Gly-Gly-Glu-Phe-Thr-Thr. Als temporäre Schutzgruppe wird die Fmoc-Gruppe, die permanten Schutzgruppen für die Seitenfunktionen auf t-Butyl/Boc-Basis, für Arg die Mtr-Gruppe und für Cys die tert.-Butylmercaptogruppen benutzt. Die Immobilisierung der C-terminalen Aminosäure erfolgt über p-Alkoxybenzylestergruppen, die an einen üblicherweise für die Peptidsynthese geeigneten polymeren Träger, vorzugsweise quervernetztem Polystyrol, gebunden sind. Der Peptidaufbau erfolgt unter repetitiver Fmoc-Abspaltung, vorzugsweise mit 20 % Piperidin in DMF (Dimethylformamid) (V/V) und Kopplung der folgenden, geschützten Aminosäure, vorzugsweise mit einem Carbodiimid in Gegenwart von HOBT. Das Aminosäurederivat wird hierzu in einem Überschuß, vorzugsweise 3fach, während 1 - 1.5 Stunden in DMF gekoppelt. Nach jedem Arbeitsschritt, Fmoc-Abspaltung bzw. Kondensationsschritt, wird das Harz mit kleinen (15 ml/g)

Portionen DMF bzw. Isopropanol je 3mal gewaschen. Die Abspaltung der erfindungsgemäßen Peptide erfolgt acidolytisch unter gleichzeitiger Freisetzung der Seitenkettenfunktionen. Gegebenenfalls freizulegende Sulfhydrylgruppen werden mit Tri-n-Butylphosphin in einem Alkohol, beispielsweise Trifluorethanol oder mit DTT in Wasser "entschützt". Die Reinigung der Peptide erfolgt über Ionenaustauschchromatographie, reversed-phase Chromatography und Gelpermeationschromatographie. Die korrekte Zusammensetzung der Peptide sowie die Peptidgehalte werden durch Aminosäureanalyse bestimmt.

Antikörper werden nach an sich bekannten Methoden hergestellt, beispielsweise indem die Peptide an ein Trägerprotein, vorzugsweise Albumin oder Keyhole Limpet Hämocyanin, bevorzugt über Thioetherbindungen, gekoppelt und damit Tiere, vorzugsweise Kaninchen, immunisiert werden. Nach einem oder mehreren Nachimmunisierungen werden die Tiere entblutet und die Rohantiseren gewonnen. Gegebenenfalls werden diese Antiseren affinitätschromatographisch an Affinitätsmaterialien chromatographiert, an die ein erfindungsgemäßes Peptid immobilisiert wurde. Darüber hinaus lassen sich nach etablierten Techniken auch monoklonale Antikörper gegen diese Peptide herstellen.

Überraschenderweise lassen sich mit diesen Antikörpern Prourokinase und Urokinase durch einen immunchemischen Nachweis, beispielsweise einen ELISA, differenzieren.

Gegenstand der Erfindung ist deshalb auch ein Mittel zur Bestimmung von Prourokinase enthaltend Antikörper gegen eines der beschriebenen Peptide.

Wird für eine solche Bestimmung ein ELISA verwendet, so wird dieser vorzugsweise als "Sandwich-Test" durchgeführt, wobei die erfindungsgemäßen Antikörper an der Festphase fixiert sind. Danach wird mit dem Analyten inkubiert und gewaschen und hierauf mit einem polyklonalen Urokinase-Antikörper, der, vorzugsweise mit POD, markiert ist, detektiert.

Weiterhin lassen sich diese Antikörper zur Reinigung der PUK einsetzen, indem die Antikörper an Polymere immobilisiert werden und PUK enthaltende Proben damit inkubiert werden. PUK kann von diesen Polymeren in gereinigter Form, vorzugsweise durch Elution mit einem sauren Puffer gewonnen werden.

Die Peptide werden zu Reinigungszwecken der Antiseren an Materialien, die als Trägermaterial zur Affinitätschromatographie geeignet sind, beispielsweise an Bromcyan aktivierte $^{R}$sepharose, gekoppelt. Weitere Trägermoleküle für die erfindungsgemäßen Peptide sind hochmolekulare Substanzen, die löslich oder auch unlöslich sind, wozu vorzugsweise Albumin, Ovalbumin, die auch glutardialdehyd-aktiviert sein können, zählen sowie Harze wie Polystyrol, derivatisierte Kieselgele und Copolymere auf Acrylsäurebasis.

## Abkürzungen

PUKProurokinase
Cys, C Cystein
Ala, A Alanin
Arg, R Arginin
Pro, P Prolin
Phe, F Phenylalanin
Lys, K Lysin
Ile, I Isoleucin
Gly, G Glycin
Glu, E Glutaminsäure
Thr, T Threonin
Gln, Q Glutamin Aminosäuren können in D- oder L-Form vorliegen, sofern jedoch nicht extra vermerkt, liegen sie in der L-Form vor.
ELISA enzym linked immuno sorbent assay
Boc t-Butoxycarbonyl
Fmoc 9-Fluorenylmethoxycarbonyl
Mtr 4-Methoxy-2,3,6-trimethylphenylsulfonyl
DMF Dimethylformamid
HOBt Hydroxybenzotriazol
DTT Dithiothreitol

## Beispiele

Beispiel 1:

Herstellung des Immunisierungsantigens

a) Synthese von Cys-Leu-Arg-Pro-Arg-Phe-Lys-Ile-Ile-Gly-Gly-Glu-Phe-Thr-Thr

1 g Fmoc-Thr (tBu)-p-alkoxybenzylesterharz wurde 2 x mit 15 ml DMF 1 min. gewaschen und die Fmoc-Gruppe mit 15 ml 20 % Piperidin/DMF (V/V) (1 x 3 min., 1 x 10 min.) abgespalten. Das Harz wurde daraufhin je 3 x mit DMF bzw. Isopropanol (je 15 ml) und 2 x mit 15 ml DMF gewaschen. Es wurden 1.5 mMol Fmoc-Aminosäure und 2.25 mMol HOBt in 15 ml gelöst zum Harz gegeben und nach Zusatz von 1.65 ml einer 1 M Diisopropylcarbodiimidlösung in Dichlormethan 1.5 h bei Raumtemperatur geschüttelt. Die Reaktion wurde mit einem Ninhydrintest auf Vollständigkeit geprüft. Danach wurde das Harz je 3 x mit DMF bzw. Isopropanol (je 15 ml) gewaschen und ein neuer Zyklus begonnen. Als letzte Aminosäure wurde eine Boc-geschützte Aminosäure benutzt. Das Harz wurde in 15 ml Trifluorethanol und 5 Tropfen Wasser aufgenommen und der pH-Wert mit N-Methylmorpholin auf 7.2 eingestellt. Es wurden 4 mMol Tri-n-Butylphosphin zugesetzt und bei Raumtemperatur über Nacht geschüttelt. Daraufhin wurde das Harz 3 x mit je 15 ml Isopropanol und Diethylether gewaschen und im Hochvakuum getrocknet. 1.9 g Harz wurden mit 1 ml Thioanisol, 1 ml Ethandithiol und 18 ml Trifluoressigsäure 3.5 h bei 35 °C gerührt, abfiltriert, das Harz mit 3 Portionen Trifluoressigsäure/Dichlormethan (1:1) gewaschen und die Filtrate in Ether kristallisiert. Das Rohpeptid wurde mit Diethylether gewaschen und getrocknet. Das Peptid wurde an $^{R}$sephadex G 25 in 0.5 % Essigsäure chromatographiert. Ausbeute: 878 mg.

100 mg dieses Produktes wurden zur Weiterreinigung an einer präparativen HPLC-Anlage an reversed-phase Material chromatographiert (0.05 mol Na-phosphatpuffer/Acetonitril, pH 2.5, Gradiententbetrieb). Der Peptidpool wurde an $^{R}$Sephadex G 25 mit 0.5 % Essigsäure entsalzt. Ausbeute: 39 mg.

b) Konjugatherstellung

20 mg KLH wurden in 0.05 mMol Natriumphosphatpuffer pH 8.0 gelöst und mit 2 mg gamma-Maleimidobuttersäurehydroxysuccimidester 1 h lang gerührt. Das Protein wurde an einer $^{R}$Sephadex G 50-Säule (2 x 30 cm) (0.1 mol Natriumphosphat, 0.5 mMol EDTA, pH 6.0) chromatographiert. Das Eluat wurde auf 5 ml konzentriert und mit 20 mg Peptid 1 h inkubiert. Nach Dialyse und Lyophilisation wurden 25 mg Peptidkonjugat erhalten.

Beispiel 2:

Immunisierung von Kaninchen

5 Kaninchen wurden mit je 2 mg Konjugat pro Tier über einen Zeitraum von 8 Wochen subcutan und intravenös immunisiert. Danach wurden die Tiere entblutet und die Antisera mit Konservierungsmittel stabilisert. Ausbeute: 920 ml.

Beispiel 3:

Reinigung der PUK-AK

Das Produkt (Beispiel 1 a) wurde in einer Konzentration von 2 mg/g BrCN-aktivierte $^{R}$Sepharose an der Matrix gebunden. 200 ml des Kaninchenserums (Beispiel 2) wurden mit 50 g des Affinitätsharzes versetzt und 18 Stunden bei RT inkubiert. Nach Abtrennung der Flüssigkeit wurde das Harz in 150 ml Puffer (PBS pH 7.2) suspendiert und in eine Säule gefüllt. Das Harz wurde anschließend mit 100 ml dieses Puffers, enthaltend 2 M NaCl, gewaschen und die Antikörper mit 200 ml einer 0.25 M Glycin-Lösung pH 2.5 eluiert. Das Eluat wurde auf pH 7.0 mit Tris eingestellt. Die Lösung wurde anschließend 18 Stunden bei 4 °C gegen PBS dialysiert.

Beispiel 4:

Nachweis von PUK im ELISA

a) Plattenvorbereitung

Die Microtiterplatten wurden mit 60 µl/Vertiefung einer Lösung, enthaltend die gereinigten Antikörper (5 µg/ml) nach Beispiel 3, beschichtet. Die Inkubationszeit betrug 18 Stunden bei RT. Die Platten wurden danach mit ca. 100 ml eines Puffers, enthaltend 50 mM Tris; 50 mM Zitronensäure pH 7.4 gewaschen.

b) Konjugatherstellung

Urokinase-Antikörper wurden nach der Methode von Vakane (Journal Histochem. Cytochem. 22, 1084 - 1091, 1974), mit POD (Peroxydase) markiert.

c) Testdurchführung

Eine PUK-Lösung (American Diagnostica) wurde auf 2400 ng/ml; 1200 ng/ml; 800 ng/ml; 400 ng/ml; 200 ng/ml; 100 ng/ml; 50 ng/ml auf 20 ng/ml mit einem Puffer, enthaltend PBS pH 7.2, 1 % BSA und 0.05 % $^{R}$Tween, verdünnt. 50 µl der verdünnten Lösungen wurden in den Vertiefungen einpipettiert. Nach der Inkubationszeit, in der sich das Antigen an die stationären Antikörper gebunden hatte (1 h bei +37 °C), wurden die Platten mit dem o.g. Puffer gewaschen. Die Peroxidase markierten UK-Antikörper wurden mit einem Puffer, enthaltend PBS pH 7.2; 0.05 % $^{R}$Tween 20, 1:800 verdünnt und 50 µl/Vertiefung einpipettiert. Die Reaktion des Konjugates mit dem Antigen wurde nach 1 h (+37 °C) mit dem Waschvorgang beendet. Eine Abfüllung chromogenes Substrat (Behringwerke), enthaltend Orthophenylendiamin, $H_2O_2$, wurde mit 10 ml eines Puffers enthaltend in 10 ml 100 mg Zitronensäure, 200 mg Dinatriumhydrogenphosphat, 8 mg Harnstoff . Wasserstoffperoxid und 1 mg Merthiolat, pH 5.0-5.4, gelöst und 50 µl/Vertiefung wurden einpipettiert. Diese Farbreaktion wurde nach 30 min. mit 0.5 M Schwefelsäure gestoppt. Die Extinktion wurde bei 492 nm im Photometer gemessen.

Tab. 1

| PUK-Konzentration | bei Extinktion 492 nm (Doppelbestimmung) | |
|---|---|---|
| | 1. Best. | 2. Best. |
| 2400 ng/ml | 1.519 | 1.513 |
| 1200 ng/ml | 0.942 | 0.976 |
| 800 ng/ml | 0.802 | 0.853 |
| 400 ng/ml | 0.532 | 0.527 |
| 200 ng/ml | 0.338 | 0.317 |
| 100 ng/ml | 0.230 | 0.189 |
| 50 ng/ml | 0.224 | 0.197 |
| 20 ng/ml | 0.218 | 0.206 |

Um die Spezifität des ELISA'S zu testen, wurden 2 Lösungen, enthaltend 400 ng/ml und 800 ng/ml PUK, mit unterschiedlichen Konzentrationen Urokinase (+20 ng/ml UK; 50 ng/ml UK; 100 ng/ml UK; 200 ng/ml UK; 400 ng/ml UK; 800 ng/ml UK; 1 µg/ml UK und 5 µg/ml UK) versetzt. Im Test zeigte sich, daß die gemessenen Werte durch die Zugabe von UK nicht beeinträchtigt werden. Diese Ergebnisse belegen die hohe Spezifität des Testes und folglich, der nach dem hier beschriebenen Verfahren gewonnenen AK gegen PUK (siehe Tab. 2).

Tab. 2

| UK Zugabe | Extinktionen bei 492 nm gemessen | | | |
|---|---|---|---|---|
| | 800 ng/ml PUK | | 400 ng/ml PUK | |
| | Doppelbestimmung | | Doppelbestimmung | |
| 0 ng/ml | 0.802 | 0.753 | 0.532 | 0.527 |
| 20 ng/ml | 0.721 | 0.743 | 0.535 | 0.479 |
| 50 ng/ml | 0.778 | 0.723 | 0.471 | 0.479 |
| 100 ng/ml | 0.750 | 0.783 | 0.533 | 0.524 |
| 200 ng/ml | 0.769 | 0.826 | 0.511 | 0.489 |
| 400 ng/ml | 0.794 | 0.764 | 0.519 | 0.506 |
| 800 ng/ml | 0.749 | 0.644 | 0.438 | 0.519 |
| 1 µg/ml | 0.729 | 0.709 | 0.449 | 0.562 |
| 5 µg/ml | 0.834 | 0.800 | 0.764 | 0.714 |

## Ansprüche

1. Peptide enthaltend 4 bis 40 Aminosäuren mit Aminosäure-Sequenzen aus der Region 140 - 180 der Prourokinase.

2. Peptide nach Anspruch 1, dadurch gekennzeichnet, daß sie die Spaltstelle 158/159 (-Lys/Ile-) der Prourokinase enthalten.

3. Peptide nach Anspruch 1, dadurch gekennzeichnet, daß sie zusätzlich Thiolgruppen, vorzugsweise am C- oder N-Terminus, enthalten.

4. Peptid nach Anspruch 1, dadurch gekennzeichnet, daß es die Aminosäuresequenz

R P R F K I I G G E F T,
L R P R F K I I G G E F T T oder
G Q K T L R P R F K I I G G E F T T I E

besitzt.

5. Peptid nach Anspruch 1, dadurch gekennzeichnet, daß es am N- oder C-Terminus Cystein mit einer freien Sulfhydrylgruppe enthält.

6. Mittel zur Bestimmung von Prourokinase enthaltend Antikörper gegen ein Peptid nach Anspruch 1.

7. Verwendung eines Peptids nach Anspruch 1 zur Herstellung von Antikörpern.

8. Verfahren zur Herstellung eines Peptids nach Anspruch 1, dadurch gekennzeichnet, daß geschützte Aminosäurederivate oder Peptidsegmente in Lösung oder an einer Festphase aneinander gekoppelt werden und durch Abspalten der Schutzgruppen sowie im Falle einer Festphase durch Abspaltung vom Trägerharz ein Peptid gemäß Anspruch 1 erhalten wird.

Patentansprüche für folgenden Vertragsstaat : ES

1. Verfahren zur Herstellung von Peptiden enthaltend 4 bis 40 Aminosäuren mit Aminosäure-Sequenzen aus der Region 140 - 180 der Prourokinase dadurch gekennzeichnet, daß geschützte Aminosäurederivate oder Peptidsegmente in Lösung oder an einer Festphase aneinander gekoppelt werden und durch Abspalten der Schutzgruppen sowie im Falle einer Festphase durch Abspaltung vom Trägerharz das Peptid erhalten wird.

2. Verfahren zur Herstellung von Peptiden nach Anspruch 1, dadurch gekennzeichnet, daß die Peptide die Spaltstelle 158/159 (-Lys/Ile-) der Prourokinase enthalten.

3. Verfahren zur Herstellung von Peptiden nach Anspruch 1, dadurch gekennzeichnet, daß die Peptide zusätzlich Thiolgruppen, vorzugsweise am C- oder N-Terminus, enthalten.

4. Verfahren zur Herstellung von einem Peptid nach Anspruch 1, dadurch gekennzeichnet, daß das Peptid die Aminosäure-Sequenz

R P R F K I I G G E F T,
L R P R F K I I G G E F T T oder
G Q K T L R P R F K I I G G E F T T I E

besitzt.

5. Verfahren zur Herstellung eines Peptids nach Anspruch 1, dadurch gekennzeichnet, daß das Peptid am N- oder C-Terminus Cystein mit einer freien Sulfhydrylgruppe enthalt.

6. Verwendung eines nach Anspruch 1 hergestellten Peptids zur Herstellung von Antikörpern.